# EUROPEAN PATENT SPECIFICATION

(11) **EP 4 094 763 B1**
(45) Date of publication and mention of the grant of the patent: **18.09.2024**
(21) Application number: 21827086.6
(22) Date of filing: 11.06.2021
(51) Int. Cl.: A61K 31/4545, A61K 31/4439, A61P 33/10, A61P 33/02, A61P 31/10, A61P 31/04, A61K 31/427, A61K 9/00, A61P 31/14, A61P 31/12, A61P 31/00, A23L 33/10

(54) **COMPOUND FOR USE IN PREVENTION AND/OR TREATMENT OF A CONDITION CAUSED BY OR ASSOCIATED WITH A CORONAVIRUS**
VERBINDUNG ZUR VERWENDUNG BEI DER VORBEUGUNG UND/ODER BEHANDLUNG EINES LEIDENS INFOLGE ODER IM ZUSAMMENHANG MIT EINEM CORONAVIRUS
COMPOSÉ POUR UNE UTILISATION DANS LA PRÉVENTION ET/OU LE TRAITEMENT D'UNE AFFECTION PROVOQUÉE PAR OU ASSOCIÉE À UN CORONAVIRUS

(30) Priority: 16.06.2020 CN 202010547186
(43) Date of publication of application: 30.11.2022
(73) Proprietor: Felicamed Biotechnology Co., Ltd., Zhuhai, Guangdong 519000 (CN)
(72) Inventor: KANG, Di, Zhuhai, Guangdong 519000 (CN); LI, Danni, Zhuhai, Guangdong 519000 (CN); LIN, Xingyu, Zhuhai, Guangdong 519000 (CN); LU, Tingting, Zhuhai, Guangdong 519000 (CN)
(74) Representative: HGF
(86) International application number: PCT/CN2021/099650
(87) International publication number: WO 2021/254265

(56) References cited:
- WO-A1-2019/090198
- WO-A1-2019/090198
- WO-A1-2019/206049
- WO-A1-2021/254118
- CN-A- 110 063 954
- CN-A- 110 215 456
- CN-A- 110 396 087
- PHILLIP MCDONAGH ET AL: "Identification and characterisation of small molecule inhibitors of feline coronavirus replication", VETERINARY MICROBIOLOGY, vol. 174, no. 3-4, 1 December 2014 (2014-12-01), NL, pages 438 - 447, XP055729240, ISSN: 0378-1135, DOI: 10.1016/j.vetmic.2014.10.030
- LI TING, ZHANG GUANGYI: "The Role of HPK1 in Ischemic Brain Injury", ACTA ACADEMIAE MEDICINAE XUZHOU, vol. 29, no. 3, 25 March 2009 (2009-03-25), pages 141 - 143, XP055881626, ISSN: 1000-2065
- ASHLEY A HORTON; BO WANG; LAUREN CAMP; MARK S PRICE; ARORA ARSHI; MATE NAGY; STEVEN A NADLER; JAMES R FAEDER; SHIRLEY LUCKHART: "The mitogen-activated protein kinome from Anopheles gambiae: identification, phylogeny and functional characterization of the ERK, JNK and p38 MAP kinases.", BMC GENOMICS, vol. 12, no. 1, 547, 23 November 2011 (2011-11-23), pages 1 - 13, XP021111596, ISSN: 1471-2164, DOI: 10.1186/1471-2164-12-574
- WANG XIAOHONG, LI JU-PI, KUO HUI-KAI, CHIU LI-LI, DEMENT GREGORY A., LAN JOUNG-LIANG, CHEN DER-YUAN, YANG CHIA-YU, HU HONGBO, TAN : "Down-regulation of B Cell Receptor Signaling by Hematopoietic Progenitor Kinase 1 (HPK1)-mediated Phosphorylation and Ubiquitination of Activated B Cell Linker Protein (BLNK).", THE JOURNAL OF BIOLOGICAL CHEMISTRY, vol. 287, no. 14, 30 March 2012 (2012-03-30), pages 11037 - 11048, XP055881632, ISSN: 0021-9258, DOI: 10.1074/jbc.M111.310946

## Description

### TECHNICAL FIELD

The present invention relates to the technical field of chemical pharmaceutics, and in particular to the a compound (4-(3-(((2-amino-5-(2-(1-methylpiperidin-4-yl)thiazol-5-yl)pyridin-3-yl)oxy)methyl) phenyl)-2-methylbut-3-yn-2-ol) for use in the prevention and/or treatment of a disease or condition caused by or associated with a pathogen infection in an animal wherein the pathogen is a coronavirus

### BACKGROUND

Viral diseases are a group of diseases doing most serious harm to animals in veterinary practice, and particularly, the prevalence of some highly infectious viral diseases often cause enormous economic and mental losses to the breeders. Viral diseases in animals usually spread quickly, feature high morbidity rate and mortality rate, and seriously endanger the health and life of animals. Moreover, some viruses can be transmitted to human beings through animals, threatening the safety of human beings.

Viral diseases in animals can be caused by a wide variety of viruses, for example, viruses being of Herpesviridae, Rhabdoviridae, Reoviridae, Poxviridae, Asfarviridae, Adenoviridae, Parvoviridae, Circoviridae, Orthomyxoviridae, Paramyxoviridae and Coronaviridae. Coronaviruses are a large family of viruses widely present in the nature and are named for their morphological similarity to crown under an electron microscope. They mainly cause respiratory diseases and can infect various mammals, such as pigs, cattle, cats, dogs, minks and camels, and various birds. For example, porcine deltacoronavirus (PDCoV) is a novel porcine enteric coronavirus that can cause diarrhea and vomiting, rapid dehydration and death of organ failure in piglets of 5-15 days old, with morbidity and mortality rates up to 50% to 100%.

Avian infectious bronchitis is an acute highly contagious respiratory infectious diseases in chicken caused by infectious bronchitis virus and is clinically characterized by dyspnea, rales, cough, mouth breathing and sneezing, a non-nephropathogenic strain that does not cause complication generally leads to a low mortality, but to reduced egg production and quality in laying hens; bovine coronavirus (BCV) is a pathogenic virus of cattle, is generally regarded as an important pathogen of neonatal calf diarrhea at present, and can cause respiratory tract infection in cattle and winter bloody dysentery in adult cattle; the epidemic diarrhea (coronavirus enteritis) in minks, foxes and raccoon dogs is caused by coronavirus except for mink parvovirus enteritis, and infected dogs, foxes, raccoon dogs and minks usually demonstrate hemorrhagic gastroenteritis symptoms and are susceptible to cluster epidemic diarrhea, showing a high spreading speed, a mortality more than 30% and a higher morbidity in animals giving birth in the year is higher than in stud animals.

Although many vaccines against viral diseases have been developed with the development of pharmaceutical technology, virus mutants impose a burden of efficacy evaluation of the vaccines against the emerging viruses as well as a financial burden, and the morbidity and mortality of viral diseases still remain high, leaving a great demand for drugs with good antiviral efficacy to the field of veterinary.

### SUMMARY

The present invention provides the compound 4-(3-(((2-amino-5-(2-(1-methylpiperidin-4-yl)thiazol-5 -yl)pyridin-3 -yl)oxy)methyl) phenyl)-2-methylbut-3-yn-2-ol (having a structure of formula I below) or a pharmaceutically acceptable salt, a solvate or a deuterated compound thereof for use the in prevention and/or treatment of a disease or condition caused by or associated with a pathogen infection in an animal wherein the pathogen is a coronavirus.

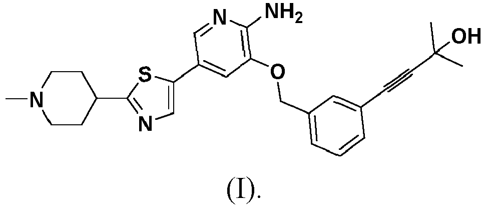

The virus according to the invention is a coronavirus, specifically, avian infectious bronchitis virus (IBV), porcine transmissible gastroenteritis virus (TGEV), porcine epidemic diarrhea virus (PEDV), porcine hemagglutinating encephalomyelitis virus (HEV), mouse hepatitis virus (MHV), turkey bluecomb virus (TCDV), bovine coronavirus (BCV), canine coronavirus (CCV), feline infectious peritonitis virus (FIPV), rat coronavirus (RCV), rat sialodacryoadenitis coronavirus (SDAV) or mink epidemic diarrhea coronavirus.

In one embodiment of the present invention, the virus described above is a feline infectious peritonitis virus.

As used herein, the term "animal" refers to a non-human animal, particularly to a vertebrate, and specifically to, e.g., a mammal (for example, pig, cattle, sheep, horse, donkey, dog, cat, rabbit, rodent, fox, racoon dog, mink, camel), fish, bird (for example, chicken, duck, goose, pigeon, quail, parrot, etc.), amphibian and reptile, unless otherwise indicated. Particularly, the animal described above is a domestic animal, i.e., an animal raised and domesticated by humans with artificially controlled reproduction for purposes such as food, labor, fur, companionship and experiment, such as an economic animal, a companion animal and a laboratory animal.

In one embodiment of the present invention, in the use described above, the animal is an economic animal, such as livestock (e.g., pig, cattle, sheep, horse, donkey, fox, racoon dog, mink and camel), poultry (chicken, duck, goose, pigeon, quail, etc.).

In one embodiment of the present invention, in the use described above, the animal is a companion animal, such as dog, cat, rabbit, rodent (e.g., guinea pig, hamster, gerbil, chinchilla and squirrel), fish, pigeon and parrot.

In one embodiment of the present invention, in the use described above, the animal is a laboratory animal, such as monkey, dog, rabbit, cat, rodent, etc.

In one embodiment of the present invention, the disease or condition described above is selected from avian infectious bronchitis, porcine transmissible gastroenteritis, porcine epidemic diarrhea, canine coronavirus disease, porcine hemagglutinating encephalomyelitis, hepatitis, encephalitis and enteritis caused by mouse hepatitis virus, turkey bluecomb, neonatal calf diarrhea, bovine blood dysentery, feline infectious peritonitis, rat sialodacryoadenitis and mink epidemic diarrhea.

In one embodiment of the present invention, the disease described above is feline infectious peritonitis.

Specifically, in the use described above, the pathogen and the animal have the corresponding definitions described above in the present invention.

In one embodiment of the present invention, the product described above is a pharmaceutical composition.

Specifically, in the pharmaceutical composition described above, the compound of formula I described above may be used as the sole active ingredient or may be used in combination with one or more additional active ingredients for the same indication or different indications, wherein the heterocyclic compound described above and the additional active ingredients may be formulated for simultaneous, separate or sequential administration.

Specifically, the pharmaceutical composition described above further comprises a pharmaceutically acceptable excipient, in particular an excipient acceptable in the field of veterinary.

Specifically, the pharmaceutical composition described above may be in any dosage form or administration form, which can be selected by those skilled in the art according to concrete circumstances. For example, the administration form can be, but is not limited to, oral, sublingual, inhalational, subcutaneous, intramuscular, intravenous, intraperitoneal, intra-organ, intranasal, intrarectal, transdermal, ocular or rectal form; the dosage form can be, but is not limited to, tablets, pills, powders, granules, capsules, lozenges, syrups, solutions, emulsions, suspensions, controlled release formulations, aerosols, films, injections, intravenous drip infusions, transdermal absorption formulations, ointments, lotions, adhesive formulations, suppositories, nasal formulations, pulmonary formulations and eye drops.

In one embodiment of the present invention, the pharmaceutical composition described above is an injection.

The various forms of the pharmaceutical composition described above can be prepared according to conventional production methods in the field of veterinary drugs.

In one embodiment of the present invention, the product described above is a functional food composition.

Specifically, in the functional food composition described above, the compound of formula I may be used as the sole active ingredient, or may be used in combination with one or more additional active ingredients.

Specifically, the functional food composition described above may further comprise an animal food excipient.

Specifically, the functional food composition described above may be in any form, such as tablets, pills, capsules, candies (e.g., tablet candies, gel candies, gum-based candies, etc.), solid beverages (e.g., powders, granules, etc.), liquid beverages and the like.

The various forms of the functional food composition described above can be prepared according to conventional production methods in the field of veterinary functional food.

In one embodiment of the present invention, the product described above is an additive, which can be added in a small amount or in a trace amount during the production, processing and use of the animal food (e.g., livestock and poultry feed, pet daily feed, pet snack and the like).

The present invention further provides a compound for use in preventing and/or treating a disease or condition caused by or associated with pathogen infection in an animal, wherein the pathogen is a coronavirus, comprising: administering to a subject an effective amount of the compound of formula I or the pharmaceutically acceptable salt, the solvate or the deuterated compound thereof described above in the present invention.

The inventor of the present invention found through experiments that the compound can effectively treat diseases (such as feline infectious peritonitis) caused by a coronavirus pathogen infection in animals, improves the survival conditions of the animals, and has better commercial value and application prospect in the field of veterinary coronavirus therapies

Phillip Mcdonagh et al. ("Identification and characterisation of small molecule inhibitors of feline coronavirus replication",VETERINARY MICROBIOLOGY,vol. 174, no. 3-4, 1 December 2014, pages 438-447) provides an overview about small molecule inhibitors of feline coronavirus replication. The document discloses that the three compounds chloroquine, mefloquine, and hexamethylene amiloride demonstrated marked inhibition of virus induced cytopathic effect (CPE) at low micromolar concentrations.

### BRIEF DESCRIPTION OF THE DRAWINGS

FIG. 1 illustrates chest X-ray images of an affected cat in Example 1 of the present invention.
FIG. 2 illustrates a curve of clinical survival in Example 2 of the present invention, wherein the administration day was taken as the first day.

### DETAILED DESCRIPTION

Unless otherwise defined, all scientific and technical terms used herein have the same meaning as commonly understood by those skilled in the art to which the present invention relates.

The term "pathogen" refers to a microorganism (including virus, chlamydia, rickettsia, mycoplasma, bacterium, spirochete, fungus and the like), a parasite (protozoon, worm and the like) or any other vector that can cause infection or diseases in humans or animals and plants. Among these, the bacterium may be, for example, a Gram-positive cocci, an enterobacterium, a vibrio, a pasteurella, a Gram-negative aerobic bacterium, a Gram-negative microaerophilic and anaerobic bacterium, a Gram-positive non-spore-forming bacillus, a Gram-positive spore-forming bacillus and mycobacterium; the fungus can be, for example, a candida, a cryptococcus and an aspergillus. In the present invention, the pathogen is a coronavirus.

The term "viral infection" refers to the process by which a virus invades the body through a variety of pathways and proliferates in susceptible host cells. The infected body may demonstrate different clinical types. The infection can be classified into apparent infection and latent infection according to the presence or absence of symptoms. An infection where the virus proliferates in host cells but no remarkable clinical symptoms are observed due to the small amount or weak toxicity of the invading virus or the strong resistance of the body is referred to as latent infection.

Although a latent infection does not demonstrate clinical symptoms, the virus proliferates *in vivo* and the body spreads new viruses to others and becomes an important source of infection. Therefore, resisting viral infection is also required for a host with latent infection. An infection where the virus proliferates in host cells and causes remarkable clinical symptoms due to the large amount or strong toxicity of the invading virus or the weak resistance of the body is referred to as latent infection.

As used herein, the term "animal" refers to a non-human animal, particularly to a vertebrate, and specifically to, e.g., a mammal (for example, pig, cattle, sheep, horse, donkey, dog, cat, rabbit, rodent, fox, racoon dog, mink, camel), fish, bird (for example, chicken, duck, goose, pigeon, quail, parrot, etc.), amphibian and reptile, unless otherwise indicated. Particularly, the animal described above is a domestic animal, i.e., an animal raised and domesticated by humans with artificially controlled reproduction for purposes such as food, labor, fur, companionship and experiment, such as an economic animal, a companion animal and a laboratory animal. The term "economic animal" refers to an animal, such as livestock, poultry and the like, that is raised for meat, milk, fur, labor or other economic purposes; the term "livestock" refers to a domestic animal that is raised and utilized by humans for breeding, and is beneficial to agricultural production; the term "poultry" refers to an avian animal that is fed by humans mainly for meat, eggs, feather or other purposes; the term "companion animal" refers to an animal raised for mental purposes (e.g., for appreciation and companion purposes) rather than economic purposes; the term "laboratory animal" refers to an animal raised for scientific application purposes.

The term "salt" is to be understood as any form of the compound according to the present invention, wherein the compound is in an ionic form, is charged and coupled with an oppositely charged ion (cation or anion), or is in a solution. Also included within this definition are quaternary ammonium salts and complexes of the molecule with other molecules and ions, particularly complexes formed by ionic interactions.

The term "solvate" is to be understood as any form of the compound of the present invention, wherein the compound is linked to another molecule (usually a polar solvent) by a non-covalent bond, particularly including hydrate and alcoholate such as methanolate. Particularly, the solvate is hydrate.

Unless otherwise specified, the compounds referred to herein may also include a deuterated compound.

The compounds described herein or the salts or solvates thereof, are in a pharmaceutically acceptable form. The term "pharmaceutically acceptable" refers to that the molecular and compositions containing the same do not produce adverse, allergic or other untoward reactions when properly administered to a subject.

The technical schemes of the present invention will be clearly and completely described below with reference to the examples of the present invention, and it is obvious that the described examples are only a part of the examples of the present invention but not all of them.

### Example 1

### 1. Methodology

A female British shorthair cat (Lele) of 2.5 kg, aged 8 month, unneutered. The cat had symptoms of increased body temperature, mental depression, decreased appetite, and increased abdominal girth in January, 2020. After 1 week, the cat was admitted. On January 18, the cat was diagnosed with feline infectious peritonitis (FIP) positive. After being treated with GS-441524 for 3 days, the cat was then administered with furosemide, Synulox^{®}, prednisolone and itraconazole (P.O., q24h, 10 mg/kg) for 1 month. The drugs were discontinued after ascites regressed. Early in April, the disease recurred, showing decreased food intake and listlessness. X-ray examination suggested hydrothorax, and PCR test showed feline coronavirus positive (the CT value was 25.11). The cat was enrolled on April 8, and was treated with the drug of the present invention at 2.0 mg/kg (SC, q24h) on days 1-14 (D1-14). Proper supportive treatments were given at the same time, including adjuvant treatment such as Moringa (a hepatoprotective drug), Lysinphirst (lysine) and Doctor Dolittle^{®} (lactoferrin).

### 2. Preparation and use

The pharmaceutically active compound described above is a pure powder have the following structure. The compound was added to a 0.9% normal saline (50 mL of endotoxin-free water and 0.45 g of NaCl), and the mixture was uniformly mixed and filtered through a filter membrane to give a clarified solution. The diluted compound was stored in a plastic-sealed sterile vial, stored in a refrigerator at 4 °C, slowly heated to room temperature before injection, and administered using a disposable syringe. The injection ranged across the back, starting from 2 cm posterior to the scapula to the middle of the lumbar spine, half of the distance of the adjacent chest to flank.

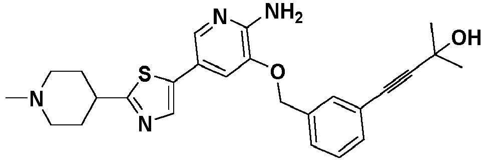

### 3. Results

The treatment was given by injection in the morning every day. Body temperature, appetite, mental state and respiratory state were measured and assessed at 15:00 pm, and urination and defecation conditions were recorded. The results are shown in Table 1.

**Table 1. Experimental results**

| **No.** | **Time** | **Body temperature Morning** | **Body temperature Afternoon** | **Mental state** | **Food intake** | **Respiratory state** | **Defecation condition** | **Notes** |
|---|---|---|---|---|---|---|---|---|
| D0 | 4-8 | | 38.5 | Listless - 3 | Decreased intake - 3 | Normal - 5 | Normal - 5 | 2.5 kg body weight at enrollment |
| D1 | 4-9 | | 38.6 | Slightly improved - 3.5 | Decreased intake - 3 | Normal | Normal | |
| D2 | 4-10 | | 38.1 | Improved - 4 | Improved - 4 | Normal | Normal | |
| D3 | 4-11 | | 38 | Good - 5 | Good - 5 | Normal | Normal | |
| D4 | 4-12 | | 38.3 | Good | Good | Normal | Normal | |
| D5 | 4-13 | | 38.3 | Good | Good | Normal | Normal | |
| D6 | 4-14 | | 38.3 | Good | Good | Normal | Normal | |
| D7 | 4-15 | | 38.1 | Good | Good | Normal | Normal | 2.75 kg body weight |
| D8 | 4-16 | | 38 | Good | Good | Normal | Normal | |
| D9 | 4-17 | | 38.2 | Good | Good | Normal | Normal | |
| D10 | 4-18 | | 38.3 | Good | Good | Normal | Normal | |
| D11 | 4-19 | | 38.4 | Good | Good | Normal | Normal | |
| D12 | 4-20 | | 38.5 | Good | Good | Normal | Normal | |
| D13 | 4-21 | 38.9 | 38.5 | Good | Good | Normal | Normal | |
| D14 | 4-22 | 38.4 | 38.4 | Good | Good | Normal | Normal | 2.75 kg body weight |

The following procedures were performed on D0, D7 and D14 after enrollment:
1 mL of serum sample was collected and frozen for multi-factor assay. The results are shown in Table 2.

**Table 2. Complete blood count results**

| Complete blood count | | Normal range | D0 | D7 | D14 |
|---|---|---|---|---|---|
| WBC | White blood cell | 5.5∼19.5 | 13.5 | 18.7 | 19.7 |
| RBC | Red blood cell | 5.0∼10.0 | 10.5 | 7.94 | 7.94 |
| HGB | Hemoglobin | 80∼150 | 117 | 94 | 91 |
| HCT | Hematocrit | 24.0∼45.0 | 37.5 | 28.9 | 29.9 |
| MCV | Mean corpuscular volume | 39.0∼55.0 | 35.9 | 36.4 | 37.7 |
| MCH | Mean corpuscular hemoglobin | 12.5∼17.5 | 11.2 | 11.8 | 11.5 |

### 3) Chest X-ray

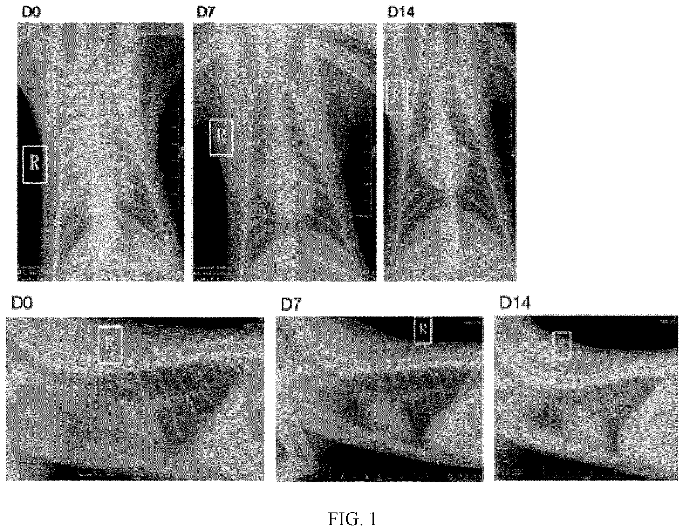

As shown in FIG. 1.

### 4) Analysis of results

The above results suggested wet FIP (in the chest), showing hydrothorax, listlessness and anorexia, which were typical clinical symptoms of feline infectious peritonitis. Because the animal had no fever symptom at enrollment, the fever alleviation effect cannot be observed during treatment, and the body temperature was always maintained at in the normal range of 38-38.5 °C throughout the treatment. After 2 weeks of treatment the cat demonstrated remarkable clinical response with improved appetite and mental state, and weight gain. The hydrothorax completely regressed since about 7 days after the treatment. The body weight increased by 9% during and after the treatment.

### Example 2

A total of 15 cats with clinically confirmed FIP were enrolled, with hydrothorax/ascites, positive CT value by PCR, and no neurological symptoms. 8 cats were allocated into the control group, and 7 cats were allocated into the treatment group.

The groups were pre-treated with GS-441524 for 3 days. For the control group, necessary supportive treatment was given on D1-D14. Group A animals were observed for 3 days, necessary supportive treatment and the drug of the present invention (2.0 mg/kg, SC, q24h) were given on D1-D14.

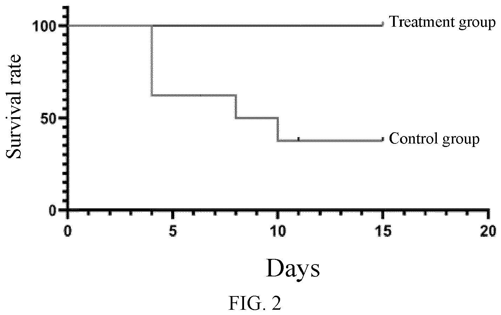

The occurrence of death or neurological symptoms was judged as clinical death, and the clinical survival curve was plotted after 14 days. The results are shown in FIG. 2. The Log Rank (Mantel-Cox) and Breslow (Generalized Wilcoxon) methods were used for validity test, and both methods indicated a significant difference (*P* < *0.05*).

## Claims

1. A compound of formula I or a pharmaceutically acceptable salt, a solvate or a deuterated compound thereof for use in prevention and/or treatment of a disease or condition caused by or associated with pathogen infection in an animal 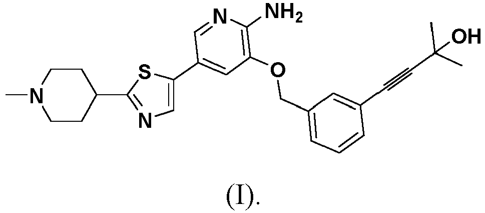 wherein the pathogen is a virus, and the virus is a coronavirus.

2. The compound, the pharmaceutically acceptable salt, the solvate or the deuterated compound thereof for the use according to claim 1, wherein the coronavirus is selected from avian infectious bronchitis virus, porcine transmissible gastroenteritis virus, porcine epidemic diarrhea virus, porcine hemagglutinating encephalomyelitis virus, mouse hepatitis virus, turkey bluecomb virus, bovine coronavirus, canine coronavirus, feline infectious peritonitis virus, rat coronavirus, rat sialodacryoadenitis coronavirus and mink epidemic diarrhea coronavirus.

3. The compound, the pharmaceutically acceptable salt, the solvate or the deuterated compound thereof for the use according to claim 1, wherein the coronavirus is feline infectious peritonitis virus.

4. The compound, the pharmaceutically acceptable salt, the solvate or the deuterated compound thereof for the use according to claim 1, wherein the animal is a domestic animal.

5. The compound, the pharmaceutically acceptable salt, the solvate or the deuterated compound thereof for the use according to claim 1, wherein the animal is livestock such as pig, cattle, sheep, horse, donkey, fox, racoon dog, mink, camel, or poultry such as chicken, duck, goose, pigeon, or quail.

6. The compound, the pharmaceutically acceptable salt, the solvate or the deuterated compound thereof for the use according to claim 1, wherein the animal is a companion animal, such as dog, cat, rabbit, rodent, fish, pigeon, or parrot.

7. The compound, the pharmaceutically acceptable salt, the solvate or the deuterated compound thereof for the use according to claim 1, wherein the animal is a laboratory animal, such as monkey, dog, rabbit, cat, or rodent.

8. The compound, the pharmaceutically acceptable salt, the solvate or the deuterated compound thereof for the use according to claim 1, wherein the disease or condition is selected from avian infectious bronchitis, porcine transmissible gastroenteritis, porcine epidemic diarrhea, canine coronavirus disease, porcine hemagglutinating encephalomyelitis, hepatitis, encephalitis and enteritis caused by mouse hepatitis virus, turkey bluecomb, neonatal calf diarrhea, bovine blood dysentery, feline infectious peritonitis, rat sialodacryoadenitis and mink epidemic diarrhea.

9. The compound, the pharmaceutically acceptable salt, the solvate or the deuterated compound thereof for the use according to claim 1, wherein the disease is feline infectious peritonitis.

## Patentansprüche

1. Präparat der Formel I oder ein pharmazeutisch akzeptables Salz, ein Solvat oder ein deuteriertes Präparat davon zur Verwendung in der Prävention und/oder Behandlung einer Erkrankung oder eines Zustands, verursacht durch oder assoziiert mit einer pathogenen Infektion in einem Tier 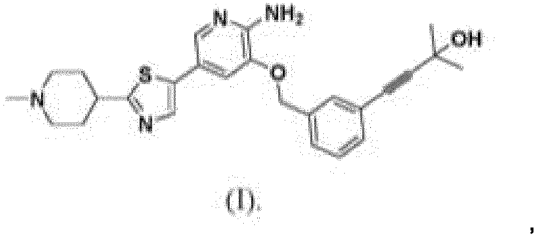 wobei das Pathogen ein Virus ist, und wobei das Virus ein Coronavirus ist.

2. Präparat, das pharmazeutisch akzeptable Salz, das Solvat oder das deuterierte Präparat davon zur Verwendung nach Anspruch 1, wobei das Coronavirus ausgewählt ist aus: Vogel-Coronavirus, übertragbarem Gastroenteritis-Coronavirus, das Schweine infiziert, porzinem epidemischem Durchfallvirus, Porcine Hemagglutinating Encephalomyelitis Virus (PHEV), Murinem Coronavirus, Coronavirus-Enteritis der Puten (Blaukammkrankheit), Bovinem Coronavirus, Canine Coronavirus, feline infektiöse Peritonitis, Ratten-Coronavirus, Ratten-Coronavirus mit Sialodacryoadenitis, Mink-Coronavirus mit epidemischem Durchfall.

3. Präparat, das pharmazeutisch akzeptable Salz, das Solvat oder das deuterierte Präparat davon zur Verwendung nach Anspruch 1, wobei das Coronavirus feline infektiöse Peritonitis ist.

4. Präparat, das pharmazeutisch akzeptable Salz, das Solvat oder das deuterierte Präparat davon zur Verwendung nach Anspruch 1, wobei das Tier ein Kleintier ist.

5. Präparat, das pharmazeutisch akzeptable Salz, das Solvat oder das deuterierte Präparat davon zur Verwendung nach Anspruch 1, wobei das Tier ein Nutztier ist, wie etwa ein Schwein, ein Rind, ein Schaf, ein Pferd, ein Esel, ein Fuchs, ein Waschbär, ein Nerz, ein Kamel oder Geflügel, wie etwa ein Huhn, eine Ente, eine Gans, eine Taube oder eine Wachtel.

6. Präparat, das pharmazeutisch akzeptable Salz, das Solvat oder das deuterierte Präparat davon zur Verwendung nach Anspruch 1, wobei das Tier ein Haustier ist, wie etwa ein Hund, eine Katze, ein Hase, ein Nagetier, ein Fisch, eine Taube oder ein Papagei.

7. Präparat, das pharmazeutisch akzeptable Salz, das Solvat oder das deuterierte Präparat davon zur Verwendung nach Anspruch 1, wobei das Tier ein Versuchstier ist, wie etwa ein Affe, ein Hund, ein Hase, eine Katze oder ein Nagetier.

8. Präparat, das pharmazeutisch akzeptable Salz, das Solvat oder das deuterierte Präparat davon zur Verwendung nach Anspruch 1, wobei die Erkrankung oder der Zustand ausgewählt ist aus: Vogel-Coronavirus, übertragbarem Gastroenteritis-Coronavirus, das Schweine infiziert, porzinem epidemischem Durchfallvirus, Canine-Coronavirus, Porcine Hemagglutinating Encephalomyelitis Virus (PHEV), Hepatitis, Encephalitis und Enteritis, verursacht durch Maus-Hepatitisvirus, Coronavirus-Enteritis der Puten (Blaukammkrankheit), Kälberdurchfall, Bovine Virus Diarrhoe, feliner infektiöser Peritonitis, Ratten-Coronavirus mit Sialodacryoadenitis, Mink-Coronavirus mit epidemischem Durchfall.

9. Präparat, das pharmazeutisch akzeptable Salz, das Solvat oder das deuterierte Präparat davon zur Verwendung nach Anspruch 1, wobei die Erkrankung feline infektiöse Peritonitis ist.

## Revendications

1. Composé de formule I ou sel pharmaceutiquement acceptable, solvate ou composé deutéré de celui-ci destiné à être utilisé dans la prévention et/ou le traitement d'une maladie ou d'un état causé par ou associé à une infection pathogène chez un animal 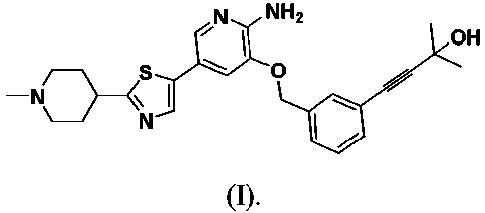 l'agent pathogène étant un virus, et le virus étant un coronavirus.

2. Composé, sel pharmaceutiquement acceptable, solvate ou composé deutéré destiné à être utilisé selon la revendication 1, le coronavirus étant choisi parmi le virus de la bronchite infectieuse aviaire, le virus de la gastro-entérite transmissible porcine, le virus de la diarrhée épidémique porcine, le virus de l'encéphalite hémagglutinante du porc, le virus de l'hépatite de la souris, le virus de l'entérite transmissible du dindon, le coronavirus bovin, le coronavirus canin, le virus de la péritonite infectieuse féline, le coronavirus du rat, le coronavirus de la sialodacryoadénite du rat et le coronavirus de la diarrhée épidémique du vison.

3. Composé, sel pharmaceutiquement acceptable, solvate ou composé deutéré destiné à être utilisé selon la revendication 1, le coronavirus étant le virus de la péritonite infectieuse féline.

4. Composé, sel pharmaceutiquement acceptable, solvate ou composé deutéré destiné à être utilisé selon la revendication 1, l'animal étant un animal domestique.

5. Composé, sel pharmaceutiquement acceptable, solvate ou composé deutéré destiné à être utilisé selon la revendication 1, l'animal étant du bétail tel qu'un porc, bovin, mouton, cheval, âne, renard, chien viverrin, vison, chameau, ou de la volaille telle qu'un poulet, canard, oie, pigeon ou caille.

6. Composé, sel pharmaceutiquement acceptable, solvate ou composé deutéré destiné à être utilisé selon la revendication 1, l'animal étant un animal de compagnie, tel qu'un chien, chat, lapin, rongeur, poisson, pigeon ou perroquet.

7. Composé, sel pharmaceutiquement acceptable, solvate ou composé deutéré destiné à être utilisé selon la revendication 1, l'animal étant un animal de laboratoire, tel qu'un singe, chien, lapin, chat ou rongeur.

8. Composé, sel pharmaceutiquement acceptable, solvate ou composé deutéré destiné à être utilisé selon la revendication 1, la maladie ou l'état étant choisi parmi la bronchite infectieuse aviaire, la gastro-entérite transmissible porcine, la diarrhée épidémique porcine, la maladie à coronavirus canin, l'encéphalite hémagglutinante du porc, l'hépatite, l'encéphalite et l'entérite causées par le virus de l'hépatite de la souris, l'entérite transmissible du dindon, la diarrhée néonatale du veau, la dysenterie sanguine bovine, la péritonite infectieuse féline, la sialodacryoadénite du rat et la diarrhée épidémique du vison.

9. Composé, sel pharmaceutiquement acceptable, solvate ou composé deutéré destiné à être utilisé selon la revendication 1, la maladie étant la péritonite infectieuse féline.
